# FASCICULE DE BREVET EUROPEEN

(11) **EP 3 083 982 B1**
(45) Date de publication et mention de la délivrance du brevet: **11.09.2019**
(21) Numéro de dépôt: 14828493.8
(22) Date de dépôt: 16.12.2014
(51) Int. Cl.: C12Q 1/04, C12Q 1/08, C12Q 1/14, G01N 33/02

(54) **PROCÉDÉ DE DÉNOMBREMENT DIFFÉRENTIEL DE BACTÉRIES LACTIQUES EN MÉLANGE DANS UN PRODUIT ALIMENTAIRE**
VERFAHREN ZUR DIFFERENTIALEN ZÄHLUNG VON MILCHSÄUREBAKTERIEN IN EINER MISCHUNG IN EINEM LEBENSMITTEL
METHOD FOR THE DIFFERENTIAL ENUMERATION OF LACTIC ACID BACTERIA IN A MIXTURE IN A FOOD PRODUCT

(30) Priorité: 16.12.2013 WO PCT/IB2013/060988
(43) Date de publication de la demande: 26.10.2016
(73) Titulaire: COMPAGNIE GERVAIS DANONE, 75009 Paris (FR)
(72) Inventeur: FOURMESTRAUX, Candice, 92350 Le Plessis Robinson (FR); COMBRISSON, Jerôme, 38100 Grenoble (FR); BOYER, Mickaël, 78210 Saint Cyr L'école (FR); GALAT, Anna, 94800 Villejuif (FR); BOUMGHAR-BOURTCHAI, Leyla, Shanghai Pudong 200122 (CN)
(74) Mandataire: Gevers & Orès
(86) Numéro de dépôt international: PCT/FR2014/053359
(87) Numéro de publication internationale: WO 2015/092258

(56) Documents cités:
- WO-A1-2008/003810
- CHEVALIER P ET AL: "X-alpha-Gal-based medium for simultaneous enumeration of bifidobacteria and lactic acid bacteria in milk", JOURNAL OF MICROBIOLOGICAL METHODS, ELSEVIER, AMSTERDAM, NL, vol. 13, no. 1, 1 avril 1991 (1991-04-01), pages 75-83, XP023699512, ISSN: 0167-7012, DOI: 10.1016/0167-7012(91)90034-N [extrait le 1991-04-01]
- W. KNEIFEL ET AL: "An X-Glu based agar medium for the selective enumeration of Lactobacillus acidophilus in yogurt-related milk products", INTERNATIONAL DAIRY JOURNAL, vol. 3, no. 3, 1 janvier 1993 (1993-01-01) , pages 277-291, XP055135226, ISSN: 0958-6946, DOI: 10.1016/0958-6946(93)90069-C
- RABIA ASHRAF ET AL: "Selective and differential enumerations of Lactobacillus delbrueckii subsp. bulgaricus, Streptococcus thermophilus, Lactobacillus acidophilus, Lactobacillus casei and Bifidobacterium spp. in yoghurt - A review", INTERNATIONAL JOURNAL OF FOOD MICROBIOLOGY, ELSEVIER BV, NL, vol. 149, no. 3, 8 juillet 2011 (2011-07-08), pages 194-208, XP028263255, ISSN: 0168-1605, DOI: 10.1016/J.IJFOODMICRO.2011.07.008 [extrait le 2011-07-21]
- MANAFI M ED - HEPERKAN DILEK ET AL: "New developments in chromogenic and fluorogenic culture media", INTERNATIONAL JOURNAL OF FOOD MICROBIOLOGY, ELSEVIER BV, NL, vol. 60, no. 2-3, 1 janvier 2000 (2000-01-01), pages 205-218, XP002177549, ISSN: 0168-1605, DOI: 10.1016/S0168-1605(00)00312-3
- N Tharmaraj ET AL: "Selective Enumeration of Lactobacillus delbrueckii ssp. bulgaricus, Streptococcus thermophilus, Lactobacillus acidophilus, Bifidobacteria, Lactobacillus casei, Lactobacillus rhamnosus, and Propionibacteria", Journal of Dairy Science, 1 juillet 2003 (2003-07-01), pages 2288-2296, XP055134253, United States DOI: 10.3168/jds.S0022-0302(03)73821-1 Extrait de l'Internet: URL:http://www.ncbi.nlm.nih.gov/pubmed/129 06045
- DANIÈLE SOHIER ET AL: "Evolution of microbiological analytical methods for dairy industry needs", FRONTIERS IN MICROBIOLOGY, vol. 5, 7 février 2014 (2014-02-07), pages 16-1, XP055176117, CH ISSN: 1664-302X, DOI: 10.3389/fmicb.2014.00016
- ZOTTA T ET AL: "A comparison of fluorescent stains for the assessment of viability and metabolic activity of lactic acid bacteria", WORLD JOURNAL OF MICROBIOLOGY AND BIOTECHNOLOGY, KLUWER ACADEMIC PUBLISHERS, DO, vol. 28, no. 3, 1 octobre 2011 (2011-10-01), pages 919-927, XP035013550, ISSN: 1573-0972, DOI: 10.1007/S11274-011-0889-X

## Description

La présente invention est relative à la différenciation et au dénombrement de souches bactériennes d'intérêt présentes en mélange dans un produit alimentaire, notamment un produit laitier.

Dans la fabrication de produits fermentés, et notamment de produits laitiers, on utilise généralement en tant que ferments des mélanges de bactéries qui peuvent comprendre des bactéries de genres différents et/ou des bactéries du même genre et d'espèces ou sous-espèces différentes, et/ou des souches différentes de bactéries de la même espèce ou sous-espèce. En particulier, les produits probiotiques comprennent habituellement, outre une ou plusieurs souches probiotiques, une ou plusieurs souches dites « technologiques », qui n'ont pas nécessairement de propriétés probiotiques, mais permettent par exemple d'améliorer la croissance des souches probiotiques et/ou de conférer au produit fini les propriétés souhaitées (goût, texture, etc.).
Afin de garantir la qualité des produits fermentés, au cours de leur fabrication, à l'issue de celle-ci, et au cours du stockage, il est nécessaire de pouvoir différencier et dénombrer spécifiquement les souches bactériennes probiotiques et technologiques présentes dans ces produits (WO 2008/003810, Chevalier et al., Journal of Microbiological Methods, 13:1, 1991, 75-83 ; Kneifel et al., International Dairy Journal, 3:3, 1993, 277-291 ; Ashraf et al., International Journal of Food microbiology, 149:3, 2011, 194-208 ; Manafi et al., International Journal of Food microbiology 60:2-3, 2000, 205-218 ; Tharmaraj et al., Journal of Dairy Science, 2003, 2288-2296 ; Zotta et al., World Journal of Microbiology and Biotechnology, 28:3, 2011, 919-927), ce qui peut poser des difficultés, notamment dans les cas où au moins 2 des souches utilisées dans le produit appartiennent à des espèces voisines d'un même genre, ou à la même espèce voire à la même sous-espèce.

Il est donc souhaitable de disposer de techniques analytiques fiables et rapides à mettre en oeuvre permettant le dénombrement différentiel de ces souches.

Les Inventeurs ont maintenant mis au point une méthode basée sur l'utilisation de milieux de culture sélectifs et/ou de conditions de cultures sélectives, associée à celle de différents substrats chromogènes.

La présente invention a en conséquence pour objet un procédé pour distinguer entre elles et dénombrer les souches de bactéries lactiques ou de Bifidobactéries d'un mélange connu, présentes à différentes quantités de populations dans un produit alimentaire, de préférence un produit laitier, fermenté en utilisant lesdites souches, caractérisé en ce qu'il comprend :
a) l'ensemencement d'aliquotes du produit alimentaire, optionnellement dilué, dans une série de boites de culture contenant un milieu gélosé chimiquement défini M1 et au moins deux substrats chromogènes produisant des colorations différentes, chacun desdits substrats étant assimilé par au moins l'une desdites souches bactériennes, et n'étant pas assimilé par au moins une autre desdites souches ;
b) optionnellement, l'ensemencement d'aliquotes du produit alimentaire, optionnellement dilué, dans une série de boites de culture contenant un milieu gélosé chimiquement défini M2 permettant la croissance des souches de bactéries lactiques présentes dans le produit à tester qui ne peuvent pas croître sur le milieu M1, et au moins un substrat chromogène assimilé par au moins l'une desdites souches bactériennes;
c) l'incubation desdites boites pendant le temps nécessaire à la formation de colonies bactériennes, et le dénombrement des colonies pour chacune des colorations observées dans chaque boîte de culture.

Au sens de l'invention, l'expression «de bactéries lactiques ou de Bifidobactéries » se réfère de préférence à l'ensemble des bactéries à Gram positif, anaérobies partiellement tolérantes à l'oxygène, ne produisant pas en général de spores, se présentant sous forme de coques ou de bâtonnets et capables de fermenter les sucres en acide lactique. Plus préférablement, les bactéries lactiques au sens de l'invention appartiennent à au moins une des familles *Aerococcaceae*, *Carnobacteriaceae*, *Enterococcaceae, Lactobacillaceae*, *Leuconostocaceae, Streptococcaceae* ou *Bifidobacteriaceae* et encore plus préférablement à un des genres *Aerococcus, Carnobacterium, Enterococcus, Lactobacillus, Lactococcus*, *Leuconostoc*, *Oenococcus*, *Pediococcus*, *Streptococcus*, *Tetragenococcus*, *Vagococcus*, *Weissella* ou *Bifidobacterium.* De manière tout particulièrement préférée, les bactéries lactiques ou les Bifidobactéries au sens de l'invention appartiennent à au moins une des espèces *Lactobacillus brevis, Lactobacillus buchneri, Lactobacillus casei, Lactobacillus curvatus, Lactobacillus delbruckei,* en particulier *L. delbruckei* supsb. *bulgaricus* ou *lactis*, *Lactobacillus diolivorans, Lactobacillus fermentum, Lactobacillus fructivorans, Lactobacillus helveticus*, *Lactobacillus hilgardii, Lactobacillus jensenii, Lactobacillus kunkeei, Lactobacillus mali, Lactobacillus nagelii*, *Lactobacillus paracasei,* en particulier *L. paracasei* subsp. *paracasei*, *Lactobacillus plantarum, Lactobacillus vini, Lactobacillus rhamnosus, Streptococcus thermophilus, Streptococcus lactis*, *Streptococcus raffinolactis, Streptococcus cremoris, Bifidobacterium adolescentis, Bifidobacterium animalis*, *Bifidobacterium bifidum, Bifidobacterium breve, Bifidobacterium infantis, Bifidobacterium lactis*, *ou Bifidobacterium longum* ; les Bifidobactéries au sens de l'invention regroupent les bactéries appartenant à la famille des *Bifidobacteriaceae,* notamment au genre *Bifidobacterium.*

On entend par « mélange connu », un mélange de souches bactériennes dont on connait la composition qualitative, c'est-à-dire la nature et les caractéristiques des différentes souches qui le constituent. Il s'agit plus spécifiquement du mélange des souches utilisé pour la fabrication du produit fermenté sur lequel porte l'analyse. Préalablement à la mise en oeuvre du procédé conforme à l'invention, ces souches auront été soumises à une analyse phénotypique visant à mettre en évidence leurs caractéristiques physiologiques et métaboliques, afin d'établir un profil phénotypique propre à chaque souche. Cette analyse peut porter notamment sur l'utilisation de différents types de sources de carbone, azote, phosphate et soufre, sur celle d'additifs nutritionnels donnés, et/ou sur la résistance à différents agents stressants (sels, pH, agents-antimicrobiens, etc.).

Le terme « milieu chimiquement défini » est utilisé ici dans son sens usuel, pour désigner un milieu de culture dont tous les composants sont complètement connus et définis. De préférence, le milieu gélosé chimiquement défini selon l'invention est un milieu de culture bactérien gélosé, plus préférablement un milieu de culture gélosé de bactéries lactiques ou de Bifidobactéries.

Comme on l'entend ici, l'expression « substrat chromogène » désigne de préférence un substrat dont l'assimilation par une bactérie produit une substance absorbant la lumière à une ou plusieurs longueurs d'onde, notamment une substance colorée. Comme l'homme du métier le comprendra bien, la ou les longueurs d'onde absorbées par la substance produite sont de préférence différentes de celle(s) éventuellement absorbée(s) par le substrat chromogène, qui peut notamment être incolore. Des substrats chromogènes utilisables pour la mise en oeuvre de la présente invention sont connus en eux-mêmes, il peut s'agir notamment 6-Chloro-3-indoxyl-beta-D-galactopyranoside (salmon Gal), 5-bromo-4-chloro-3-indolyl-beta-D-glucopyranoside (X-glu), 5-Bromo-4-Chloro-3-Indolyl-Beta-D-Galactopyranoside (X-Gal).

Le milieu gélosé d'une partie des boites de cultures peut comprendre en outre au moins un additif favorisant sélectivement la croissance d'au moins l'une desdites souches bactériennes, et/ou le milieu gélosé d'une partie des boites de cultures peut comprendre en outre au moins un additif inhibant sélectivement la croissance d'au moins l'une desdites souches bactériennes.

Également, une partie des boîtes de culture peut être incubée dans des conditions sélectives favorables à la croissance d'au moins l'une desdites souches bactériennes, et une autre partie desdites boites de culture peut être incubée dans des conditions sélectives différentes, favorables à la croissance d'au moins une autre desdites souches bactériennes.

Le procédé conforme à l'invention est particulièrement approprié à l'analyse de produits où au moins deux des souches bactériennes présentes appartiennent à la même espèce et sous-espèce.

De préférence, au moins une des souches bactériennes présentes dans le produit à tester appartient au genre *Lactobacillus* et/ou au moins une des souches bactériennes présentes dans le produit à tester appartient au genre *Streptococcus.*

Selon un mode de mise en oeuvre particulier de la présente invention, le produit à tester contient au moins une souche de *Lactobacillus paracasei* subsp. *paracasei*, au moins une souche de *Lactobacillus delbrueckii* subsp. *bulgaricus,* et au moins une souche de *Streptococcus thermophilus.* Avantageusement, il contient en outre au moins une souche de *Lactobacillus rhamnosus.*

Dans le cadre de ce mode de mise en oeuvre, on utilise, pour dénombrer les bactéries des espèces *Lactobacillus paracasei* subsp. *paracasei, Lactobacillus rhamnosus,* et *Streptococcus thermophilus* un milieu M1 dont la composition est la suivante : agar : 15 g/L ; tryptone : 2,5 g/L ; peptone pepsique de viande : 2,5 g/L ; peptone papainique de soja : 5 g/L ; glycérophosphate de sodium : 19 g/L ; lactose : 5 g/L ; extrait de levure : 2,5 g/L ; extrait de viande : 5 g/L ; sulfate de magnésium : 0,25 g/L ; acide ascorbique : 0,5 g/L ; 6-Chloro-3-indoxyl-beta-D-galactopyranoside (salmon Gal) : 0,2 g/L ; 5-bromo-4-chloro-3-indolyl-beta-D-glucopyranoside (X-glu) : 0,1 g/L.

Pour dénombrer les bactéries de l'espèce *Lactobacillus delbrueckii* subsp. *bulgaricus* on utilise un milieu M2 dont la composition est la suivante : polypeptone : 10 g/L ; extrait de levure : 5 g/L ; extrait de viande : 10 g/L ; phosphate dipotassique : 2 g/L ; acétate de sodium : 5 g/L ; citrate d'ammonium : 2 g/L ; sulfate de magnésium : 0,2 g/L ; sulfate de manganèse : 0,05 g/L ; agar : 15 g/L ; 5-Bromo-4-Chloro-3-Indolyl-Beta-D-Galactopyranoside (X-Gal) : 0,15 g/L.

Avantageusement, pour dénombrer les bactéries des espèces *Lactobacillus paracasei* subsp. *paracasei*, *Lactobacillus rhamnosus,* et *Streptococcus thermophilus* :
- une première partie des boites contenant le milieu M1 ne comprend pas d'additif, une seconde partie desdites boites comprend, en tant qu'additif, de la vancomycine, et une troisième partie desdites boites comprend, en tant qu'additif, du rhamnose ; et/ou
- les boites ensemencées sont incubées pendant environ 48 heures en atmosphère contrôlée contenant de 6 à 16% d'O₂ et de 2 à 10% de CO₂, une partie desdites boîtes étant incubées à environ 37°C, et une autre partie à environ 44°C.

De préférence, pour dénombrer les bactéries de l'espèce *Lactobacillus delbrueckii* subsp. *bulgaricus*, les boites de milieu M2 ensemencées sont incubées pendant environ 48 heures à environ 47°C en atmosphère contrôlée contenant moins de 1% d'O₂ et au moins 13% de CO₂.

La présente invention sera mieux comprise à l'aide du complément de description qui va suivre, qui se réfère à des exemples non-limitatifs illustrant la mise en oeuvre d'un procédé conforme à l'invention pour différencier différentes souches de bactéries lactiques, et dénombrer des bactéries de chacune de ces souches dans des produits laitiers fermentés.

### EXEMPLE: DENOMBREMENT DIFFERENTIEL DE BACTERIES LACTIQUES DANS DES PRODUITS LAITIERS FERMENTES.

### Matériels et méthodes :

### Composition des produits testés

### Produit 1 :

Le produit 1 est un produit fermenté contenant 5 souches bactériennes appartenant à trois espèces, à savoir :
- 1 souche probiotique de *L. casei* subsp. *paracasei* (ci-après dénommée *Lactobacillus paracasei* souche 1) ;
- 3 souches technologiques de *S. thermophilus ;*
- 1 souche technologique de *L. delbrueckii* subsp. *Bulgaricus.*

Les charges bactériennes théoriques attendues en début de vie (J4) et en fin de vie (J35) du produit sont indiquées dans le Tableau I ci-dessous.

**Tableau I**

| | Charge bactérienne théorique (en bactéries/ml) | |
|---|---|---|
| | J4 | J35 |
| *Lactobacillus paracasei* souche 1 | 2.10⁸ | 2.10⁸ |
| *3 Streptococcus thermophilus* | > 10⁸ | > 10⁸ |
| *Lactobacillus bulgaricus* | *1.10⁷* | *10¹ à 10⁴* |

### Produit 2 :

Le produit 2 est un produit fermenté contenant 7 souches bactériennes appartenant à trois espèces, à savoir :
- 2 souches probiotiques de *L. casei* subsp. *paracasei* (*Lactobacillus paracasei* souche 1 et *Lactobacillus paracasei* souche 2) ;
- 1 souche probiotique de *L. rhamnosus* ;
- 3 souches technologiques de *S. thermophilus* ;
- 1 souche technologique de *L. delbrueckii* subsp. *Bulgaricus.*

Les charges bactériennes théoriques attendues en début (J4) et en fin de vie (J35) du produit sont indiquées dans le Tableau II ci-dessous.

**Tableau II**

| | Charge bactérienne théorique (en bactéries/ml) | |
|---|---|---|
| | J4 | J35 |
| *Lactobacillus paracasei* souche 1 et souche 2 | 2 à 3.10⁸ | 2 à 3.10⁸ |
| *Lactobacillus rhamnosus* | 1.10⁸ | 1.10⁸ |
| *3 Streptococcus thermophilus* | 1.10⁸ | 1.10⁸ |
| *Lactobacillus bulgaricus* | 1.10⁷ | 10¹ à 10⁴ |

### Activités enzymatiques des souches :

Les différentes souches ont été testées pour leurs activités β-Glucosidase et β-Galactosidase.

Les résultats sont indiqués dans le Tableau III ci-dessous :

**Tableau III**

| Souche | β-Glucosidase | β-Galactosidase |
|---|---|---|
| *Lactobacillus rhamnosus* | + | + |
| *Lactobacillus paracasei 1* | + | - |
| *Lactobacillus paracasei 2* | - | - |
| *Streptococcus thermophilus 1* | - | + |
| *Streptococcus thermophilus 2* | - | + |
| *Streptococcus thermophilus 3* | - | + |
| *Lactobacillus bulgaricus* | - | + |

### Dénombrement des bactéries dans les produits 1 et 2 :

### Milieux et conditions de culture :

Les milieux et conditions de culture actuellement utilisés pour le dénombrement des souches bactériennes présentes dans les produits 1 et 2 sont résumés respectivement dans les tableaux IV et V ci-dessous. Ces milieux et conditions de culture de référence ont été utilisés à titre de témoins.

**Tableau IV**

| Souche recherchée | Milieu | Température | Durée | Atmosphère |
|---|---|---|---|---|
| *Lactobacillus paracasei* 1 | MRS + vancomycine (1µg/mL) | 37°C | 48 heures | CO₂ |
| *Streptococcus sp* | M17 | 44°C | 48 heures | Aérobie |
| *Lactobacillus bulgaricus* | MRS acide | 50°C | 48 heures | Anaérobie |

**Tableau V**

| Souche recherchée | Milieu | Température | Durée | Atmosphère |
|---|---|---|---|---|
| *Lactobacillus rhamnosus* | MRS + vancomycine (1µg/mL) | 44°C | 48 heures | CO₂ |
| *Lactobacillus rhamnosus* + *Lactobacillus paracasei 1* + *Lactobacillus paracasei* 2 | MRS + vancomycine (1µg/mL) | 37°C | 48 heures | CO₂ |
| *Streptococcus sp* | M17 | 44°C | 48 heures | Aérobie |
| *Lactobacillus bulgaricus* | MRS acide | 50°C | 48 heures | Anaérobie |

Les milieux MRS, MRS acide, et M17 sont des milieux commerciaux classiques (AES CHEMUNEX).

Pour les cultures sous atmosphère contrôlée, des systèmes générateurs de gaz GASPAK® ont été utilisés, pour obtenir les conditions suivantes :
- anaérobiose : %O₂ < 1% ; %CO₂ ≥ 13%
- CO₂ : %CO₂ > 2,5%
- Micro-aérobiose : 6% <%O₂<16% ; 2% <%CO₂< 10%
- aérobiose : air ambiant

Deux milieux chromogènes, dénommés ci-après M1 et M2 ont été préparés : la composition de ces milieux est indiquée respectivement dans les Tableaux VI et VII ci-dessous :

**Tableau VI**

| Milieu M1 | |
|---|---|
| Constituant | Concentration (en g/L) |
| Agar | 15 |
| Tryptone | 2,5 |
| Peptone pepsique de viande | 2,5 |
| Peptone papainique de soja | 5 |
| Glycérophosphate de sodium | 19 |
| Lactose | 5 |
| Extrait de levure | 2,5 |
| Extrait de viande | 5 |
| Sulfate de magnésium | 0,25 |
| Acide ascorbique | 0,5 |
| 6-Chloro-3-indoxyl-beta-D-galactopyranoside (salmon Gal) | 0,2 |
| 5-bromo-4-chloro-3-indolyl-beta-D-glucopyranoside (X-glu) | 0,1 |

**Tableau VII**

| Milieu M2 | |
|---|---|
| Constituant | concentration (en g/L) |
| Polypeptone | 10 |
| Extrait de levure | 5 |
| Extrait de viande | 10 |
| Phosphate dipotassique | 2 |
| Acétate de sodium | 5 |
| Citrate d'ammonium | 2 |
| Sulfate de magnésium | 0,2 |
| Sulfate de manganèse | 0,05 |
| Agar | 15 |
| 5-Bromo-4-Chloro-3-Indolyl-Bta-D-Galactopyranoside (X-Gal) | 0,15 |

Les essais ont été effectués avec 3 lots différents de chacun des milieux M1 et M2.

Les conditions de cultures utilisées pour les produits 1 et 2 sont respectivement indiquées dans les Tableaux VIII et IX ci-dessous :

**Tableau VIII**

| Souche recherchée | Milieu | Température | Durée | Atmosphère |
|---|---|---|---|---|
| *Lactobacillus paracasei* 1 + *Streptococcus* sp | M1 | 37°C | 48 heures | Micro-Aérobie |
| *Lactobacillus bulgaricus* | M2 | 47°C | 48 heures | Anaérobie |

**Tableau IX**

| Souche recherchée | Milieu | Température | Durée | Atmosphère |
|---|---|---|---|---|
| *Lactobacillus paracasei* 1 | M1 + rhamnose (10 g/L) | 37°C | 48 heures | Micro-Aérobie |
| *Lactobacillus paracasei* 2 | M1 + vancomycine (1µg/mL) | 37°C | 48 heures | Micro-Aérobie |
| *Lactobacillus rhamnosus* + *Streptococcus* sp | M1 | 44°C | 48 heures | Micro-Aérobie |
| *Lactobacillus bulgaricus* | M2 | 47°C | 48 heures | Anaérobie |

### Protocole opératoire :

Des échantillons ont été prélevés à deux phases de la vie des produits : début de vie, et fin de vie.

Pour le produit 1, les prélèvements de début de vie ont été effectués à 4, 5, et 6 jours après la fabrication du produit fermenté, et les prélèvements de fin de vie ont été effectués à 31, 32, et 33 jours après la fabrication ; pour le produit 2, les prélèvements de début de vie ont été effectués à 7, 8, et 9 jours après la fabrication, et les prélèvements de fin de vie ont été effectués à 28, 29, et 30 jours après la fabrication.

Pour chaque prise d'essai, une gamme de dilutions au 1/10 a été effectuée en tubes tryptone sel. Trois dilutions, choisies en fonction des populations théoriques, (cf. Tableaux I et II) ont été ensemencées sur chacun des milieux utilisés. Pour chaque dilution, 1 mL de dilution a été ensemencé dans 1 boite de Pétri, et 15 ml du milieu utilisé ont été coulés dans la boite.

Les boites ont été incubées dans les conditions et pendant les durées indiquées aux Tableaux IV, V, VIII, et IX.

### Résultats :

### Identification des souches :

Sur le milieu M1 :
   - à 44°C, la souche de *Lactobacillus rhamnosus* produit des colonies de couleur bleue, et les souches de *Streptococcus thermophilus* produisent des colonies de couleur magenta ; les souches 1 et 2 de *Lactobacillus paracasei* et la souche de *Lactobacillus bulgaricus* ne croissent pas.
   - à 37°C avec ajout de rhamnose, la souche de *Lactobacillus rhamnosus* et la souche 2 de *Lactobacillus paracasei* produisent des colonies incolores ; la souche 1 de *Lactobacillus paracasei* produit des colonies de couleur turquoise, et les souches de *Streptococcus thermophilus* produisent des colonies de couleur magenta. La souche de *Lactobacillus bulgaricus* ne croît pas.
   - à 37°C avec ajout de vancomycine, la souche de *Lactobacillus rhamnosus* produit des colonies bleues ; la souche 1 de *Lactobacillus paracasei* produit des colonies de couleur turquoise, et la souche 2 de *Lactobacillus paracasei* produit des colonies incolores ; les souches de *Streptococcus thermophilus* et la souche de *Lactobacillus bulgaricus* ne croissent pas.
Sur le milieu M2 :
   - la souche de *Lactobacillus rhamnosus* produit des colonies incolores, et la souche de *Lactobacillus bulgaricus* produit des colonies bleues. Les souches de *Streptococcus thermophilus* et les souches 1 et 2 de *Lactobacillus paracasei* ne croissent pas.

Ces résultats sont résumés dans le Tableau X ci-dessous :

**Tableau X**

| Nom de la souche | Milieu M1 | | | Milieu M2 |
|---|---|---|---|---|
| | Incubation à 44°C | Ajout de rhamnose et incubation à 37°C | Ajout de vancomycine et incubation à 37°C | |
| *Lactobacillus rhamnosus* | Bleu | Incolore | Bleu | Incolore |
| *Lactobacillus paracasei* 1 | Absence de croissance | Turquoise | Turquoise | Absence de croissance |
| *Lactobacillus paracasei 2* | Absence de croissance | Incolore | Incolore | Absence de croissance |
| *Streptococcus thermophilus (souches 1, 2, et 3)* | Magenta | Magenta | Absence de croissance | Absence de croissance |
| *Lactobacillus bulgaricus* | Absence de croissance | Absence de croissance | Absence de croissance | Bleu |
| Souche(s) identifiée(s) | *Lactobacillus rhamnosus* et *Streptococcus thermophilus* | *Lactobacillus paracasei 1* | *Lactobacillus paracasei 2* | *Lactobacillus bulgaricus* |

### Produit 1 :

Le milieu M1 permet donc de dénombrer *Lactobacillus paracasei* et *Streptococcus thermophilus,* et le milieu M2 de dénombrer *Lactobacillus bulgaricus.*

### Produit 2 :

Le milieu M1 permet donc de dénombrer :
- à 44°C : *Lactobacillus rhamnosus* et *Streptococcus thermophilus ;*
- avec ajout de rhamnose à 37°C : la souche 1 de *Lactobacillus paracasei ;*
- avec ajout de vancomycine à 37°C : la souche 2 de *Lactobacillus paracasei.*

Le milieu M2 permet de dénombrer *Lactobacillus bulgaricus.*

### Dénombrement des bactéries :

### Produit 1 :

### Début de vie :

Les résultats sont illustrés dans le Tableau XI.

**Tableau XI**

| Espèce | | Milieu de référence (témoin) | Lot 1 | Lot 2 | Lot 3 |
|---|---|---|---|---|---|
| *L.paracasei 1* | Charge bactérienne | 5,4.10⁸ | 6,1.10⁸ | 5,7.10⁸ | 6,2.10⁸ |
| | Différence vs le témoin (en log) | | + 0,06 | + 0,03 | + 0,07 |
| *S.thermophilus* | Charge bactérienne | 6,4.10⁸ | 5,3.10⁸ | 6,8.10⁸ | 5,5.10⁸ |
| | Différence vs le témoin (en log) | | - 0,08 | + 0,03 | - 0,07 |
| *L.bulgaricus* | Charge bactérienne | 1,6.10⁶ * | 3.10⁶ | 3.10⁶ | 2,8.10⁶ |
| | Différence vs le témoin (en log) | | + 0,27 | + 0,26 | 0,23 |

La variabilité de la méthode de dénombrement est la suivante :
- Sur milieu M1 : entre 5,7.10⁸ UFC/ml et 6,2.10⁸ UFC/ml pour *L.paracasei 1*, entre 5,3.10⁸ UFC/ml et 6,8.10⁸ UFC/ml pour *S. thermophilus,*
- Sur milieu M2: entre 2,8.10⁶ UFC/ml et 3.10⁶ UFC/ml pour *L.bulgaricus.*

Des difficultés de dénombrement de *L. bulgaricus* sur le milieu de référence, MRS acide, sont à noter :
- jour 5 : différence d'un log versus le milieu M2 et le dénombrement de J4,
- jour 6 : absence de croissance.

### Fin de Vie :

Les résultats sont illustrés dans le Tableau XII.

**Tableau XII**

| Espèce | | Milieu de référence (témoin) | Lot 1 | Lot 2 | Lot 3 |
|---|---|---|---|---|---|
| *L.paracasei 1* | Charge bactérienne | 4,5.10⁸ | 4,1.10⁸ | 4.10⁸ | 4,5.10⁸ |
| | Différence vs le témoin (en log) | | - 0,04 | - 0,05 | - 0,01 |
| *S.thermophilus* | Charge bactérienne | 1,8.10⁸ | 1,9.10⁸ | 1,9.10⁸ | 2,2.10⁸ |
| | Différence vs le témoin (en log) | | + 0,02 | + 0,02 | + 0,08 |
| *L.bulgaricus* | Charge bactérienne | Absence | Absence | Absence | Absence |
| | Différence vs le témoin (en log) | | N.A. | N.A. | N.A. |

Le milieu M1 permet de discriminer de façon efficace *Lactobacillus paracasei* et *Streptococcus thermophilus* présents dans le produit. Les dénombrements de *L. paracasei* varient entre 4.10⁸ UFC/ml et 4,5.10⁸ UFC/ml entre les trois lots et sur trois jours d'analyse. La charge de *S.thermophilus* varie entre 1,9.10⁸ UFC/ml et 2,2.10⁸ UFC/ml.

Ces essais mettent en outre en évidence l'absence de *L. bulgaricus* dans le produit à ce stade de vie que ce soit sur le milieu référence ou le milieu M2.

### Produit 2 :

### Début de vie :

Les résultats sont illustrés dans le Tableau XIII.

**Tableau XIII**

| Espèce | | Milieu de référence (témoin) | 1 | Lot 2 | Lot 3 |
|---|---|---|---|---|---|
| *L.paracasei* 1 et 2 | Charge *1* | 2.10⁸ | 1,6.10⁸ | 1,8.10⁸ | 1,7.10⁸ |
| | Charge bactérienne *L.paracasei* 2 | | 2,3.10⁷ | 3.10⁷ | 2,8.10⁷ |
| | Différence vs le témoin (en log) | | - 0,02 | + 0,03 | 0,00 |
| *S.thermophilus* | Charge bactérienne | 8,9.10⁸ | 8,7.10⁸ | 9,7.10⁸ | 9,8.10⁸ |
| | Différence vs le témoin (en log) | | - 0,01 | + 0,01 | + 0,04 |
| *L.rhamnosus* | Charge bactérienne | 2,3.10⁸ | 2,3.10⁸ | 2,3.10⁸ | 2,3.10⁸ |
| | Différence vs le témoin (en log) | | 0,00 | 0,00 | 0,00 |
| *L.bulgaricus* | Charge bactérienne | 2,5.10⁵ | 4,6.10⁵ | 2,6.10⁵ | 3,1.10⁵ |
| | Différence vs le témoin (en log) | | + 0,27 | + 0,03 | 0,10 |

La variabilité de la méthode de dénombrement est la suivante :
- Sur milieu M1 : entre 1,6.10⁸ UFC/ml et 1,8.10⁸ UFC/ml pour *L.paracasei* 1, entre 2, 3.10⁷ UFC/ml et 3.10⁷ UFC/ml pour *L.paracasei* 2, entre 8,7.10⁸ UFC/ml et 9,8.10⁸ UFC/ml pour *S.thermophilus*, 2,3.10⁸ UFC/ml pour les trois lots pour *L.rhamnosus*
- Sur milieu M2: entre 2,6.10⁵ UFC/ml et 5.10⁵ UFC/ml pour *L.bulgaricus.*

### Fin de Vie :

Les résultats sont illustrés dans le Tableau XIV.

**Tableau XIV**

| Espèce | | Milieu de référence (témoin) | Lot 1 | Lot 2 | Lot 3 |
|---|---|---|---|---|---|
| *L.paracasei 1 et 2* | Charge bactérienne *L.paracasei 1* | 2,1.10⁸ | 1,6.10⁸ | 1,5.10⁸ | 1,6.10⁸ |
| | Charge bactérienne *L.paracasei* 2 | | 1,4.10⁷ | 2,2.10⁷ | 2,2.10⁷ |
| | Différence vs le témoin (en log) | | - 0,07 | - 0,08 | - 0,05 |
| *S.thermophilus* | Charge bactérienne | 7,3.10⁸ | 6,8.10⁸ | 6,6.10⁸ | 7,5.10⁸ |
| | Différence vs le témoin (en log) | | - 0,03 | - 0,04 | + 0,01 |
| *L.rhamnosus* | Charge bactérienne | 1,8.10⁸ | 2,0.10⁸ | 2,0.10⁸ | 2,0.10⁸ |
| | Différence vs le témoin (en log) | | + 0,03 | + 0,04 | + 0,04 |
| *L.bulgaricus* | Charge bactérienne | Absence | Absence | Absence | Absence |
| | Différence vs le témoin (en log) | | N.A. | N.A. | N.A. |

Les dénombrements sur les trois lots de milieux M1 varient entre 1,5.10⁸ UFC/ml et 1,6.10⁸ UFC/ml pour *L.paracasei* 1, entre 1,4.10⁷ UFC/ml et 2,2.10⁷ UFC/ml pour *L.paracasei* 2, entre 6,6.10⁸ UFC/ml et 7,5.10⁸ UFC/ml pour *S.thermophilus*, 2,0.10⁸ UFC/ml pour les trois lots pour *L.rhamnosus.*

Les essais mettent en outre en évidence l'absence de *L. bulgaricus* dans le produit à ce stade de vie que ce soit sur le milieu référence ou le milieu M2.

L'utilisation de la méthode décrite ci-dessus permet de discriminer les trois espèces bactériennes présentes dans le produit 1 et les quatre présentes dans le produit 2.

## Revendications

1. Procédé pour distinguer entre elles et dénombrer des souches de bactéries lactiques ou Bifidobactéries en mélange connu, présentes en différentes quantités de population dans un produit laitier, **caractérisé en ce qu'**il comprend :
a) l'ensemencement d'aliquotes dudit produit alimentaire dans une série de boites de culture contenant chacune un milieu gélosé chimiquement défini M1 et au moins deux substrats chromogènes produisant des colorations différentes, chacun desdits substrats étant assimilé par au moins l'une desdites souches bactériennes, et n'étant pas assimilé par au moins une autre desdites souches ; et
b) optionnellement, l'ensemencement d'aliquotes dudit produit alimentaire dans une série de boites de culture contenant un milieu gélosé chimiquement défini M2 permettant la croissance des souches de bactéries lactiques présentes dans le produit à tester qui ne peuvent pas croître sur le milieu M1, et au moins un substrat chromogène assimilé par au moins l'une desdites souches bactériennes; et
c) l'incubation desdites boites pendant le temps nécessaire à la formation de colonies bactériennes, et le dénombrement des colonies pour chacune des colorations observées dans chaque boîte de culture.

2. Procédé selon la revendication 1, **caractérisé en ce que** le milieu gélosé d'une partie des boites de cultures comprend en outre au moins un additif favorisant sélectivement la croissance d'au moins l'une desdites souches bactériennes, et/ou le milieu gélosé d'une partie des boites de cultures comprend en outre au moins un additif inhibant sélectivement la croissance d'au moins l'une desdites souches bactériennes,

3. Procédé selon une quelconque des revendications 1 ou 2, **caractérisé en ce qu'**une partie desdites boîtes de culture est incubée dans des conditions sélectives favorables à la croissance d'au moins l'une desdites souches bactériennes, et une autre partie des boites de culture est incubée dans des conditions sélectives différentes, favorables à la croissance d'au moins une autre desdites souches bactériennes

4. Procédé selon une quelconque des revendications 1 à 3, **caractérisé en ce qu'**au moins deux des souches bactériennes présentes dans le produit à tester appartiennent à la même espèce et sous-espèce.

5. Procédé selon une quelconque des revendications 1 à 4, **caractérisé en ce qu'**au moins une des souches bactériennes présentes dans le produit à tester appartient au genre *Lactobacillus* et/ou au moins une des souches bactériennes présentes dans le produit à tester appartient au genre *Streptococcus.*

6. Procédé selon une quelconque des revendications 1 à 5, **caractérisé en ce que** le produit à tester contient au moins une souche de *Lactobacillus paracasei* subsp. *paracasei,* au moins une souche de *Lactobacillus delbrueckii* subsp. *bulgaricus*, et au moins une souche de *Streptococcus thermophilus.*

7. Procédé selon la revendication 6 **caractérisé en ce que** le produit à tester contient en outre au moins une souche de *Lactobacillus rhamnosus.*

8. Procédé selon une quelconque des revendications 6 ou 7, **caractérisé en ce que** pour dénombrer les bactéries des espèces *Lactobacillus paracasei* subsp. *paracasei*, *Lactobacillus rhamnosus,* et *Streptococcus thermophilus* on utilise un milieu M1 dont la composition est la suivante : agar : 15 g/L ; tryptone : 2,5 g/L ; peptone pepsique de viande : 2,5 g/L ; peptone papainique de soja : 5 g/L ; glycérophosphate de sodium : 19 g/L ; lactose : 5 g/L ; extrait de levure : 2,5 g/L ; extrait de viande : 5 g/L ; sulfate de magnésium : 0,25 g/L ; acide ascorbique : 0,5 g/L ; 6-Chloro-3-indoxyl-beta-D-galactopyranoside (salmon Gal) : 0,2 g/L ; 5-bromo-4-chloro-3-indolyl-beta-D-glucopyranoside (X-glu) : 0,1 g/L.

9. Procédé selon une quelconque des revendications 7 ou 8, **caractérisé en ce que** pour dénombrer les bactéries de l'espèce *Lactobacillus delbrueckii bulgaricus* on utilise un milieu M2 dont la composition est la suivante : polypeptone : 10 g/L ; extrait de levure : 5 g/L ; extrait de viande : 10 g/L ; phosphate dipotassique : 2 g/L ; acétate de sodium : 5 g/L ; citrate d'ammonium : 2 g/L ; sulfate de magnésium : 0,2 g/L ; sulfate de manganèse : 0,05 g/L ; agar : 15 g/L ; 5-Bromo-4-Chloro-3-Indolyl-Beta-D-Galactopyranoside (X-Gal) : 0,15 g/L.

10. Procédé selon la revendication 8, **caractérisé en ce que** pour dénombrer les bactéries des espèces *Lactobacillus paracasei* subsp. *paracasei*, *Lactobacillus rhamnosus,* et *Streptococcus thermophilus,* une première partie des boites contenant le milieu M1 ne comprend pas d'additif, une seconde partie desdites boites comprend, en tant qu'additif, de la vancomycine, et une troisième partie desdites boites comprend, en tant qu'additif, du rhamnose.

11. Procédé selon une quelconque des revendications 7 à 10, **caractérisé en ce que** pour dénombrer les bactéries des espèces *Lactobacillus paracasei* subsp. *paracasei*, *Lactobacillus rhamnosus,* et *Streptococcus thermophilus*, les boites ensemencées sont incubées pendant environ 48 heures en atmosphère contrôlée contenant de 6 à 16% d'O₂ et de 2 à 10% de CO₂, une partie desdites boîtes étant incubée à environ 37°C, et une autre partie à environ 44°C.

12. Procédé selon une quelconque des revendications 7 à 10, **caractérisé en ce que** pour dénombrer les bactéries de l'espèce *Lactobacillus delbrueckii bulgaricus,* les boites ensemencées sont incubées pendant environ 48 heures à environ 47°C en atmosphère contrôlée contenant moins de 1% d'O₂ et au moins 13%.de CO₂.

## Patentansprüche

1. Verfahren zum Unterscheiden voneinander und Zählen von Milchsäurebakterien- oder Bifidobakterienstämmen in bekanntem Gemisch, die in unterschiedlichen Bevölkerungsmengen in einem Molkereiprodukt vorhanden sind, **dadurch gekennzeichnet, dass** es umfasst:
a) das Beimpfen von Teilproben des Nahrungsmittelprodukts in einer Reihe von Kulturbehältern, die jeweils ein chemisch definiertes Agarmedium M1 und mindestens zwei chromogene Substrate enthalten, die unterschiedliche Färbungen erzeugen, wobei jedes der Substrate durch den mindestens einen der Bakterienstämme assimiliert ist und durch mindestens einen anderen der Stämme nicht assimiliert ist; und
b) wahlweise das Beimpfen von Teilproben des Nahrungsmittelprodukts in einer Reihe von Kulturbehältern, die ein chemisch definiertes Agarmedium M2, das das Wachstum der im zu prüfenden Produkt vorhandenen Milchsäurebakterienstämme ermöglicht, die nicht auf dem Medium M1 wachsen können, und mindestens ein chromogenes Substrat, das durch den mindestens einen der Bakterienstämme assimiliert ist, enthalten; und
c) die Inkubation der Behälter während der Zeit, die für die Bildung von Bakterienkolonien erforderlich ist, und das Zählen der Kolonien für jede der in jedem Kulturbehälter beobachteten Färbungen.

2. Verfahren nach Anspruch 1, **dadurch gekennzeichnet, dass** das Agarmedium eines Teils der Kulturbehälter weiter mindestens einen Zusatz umfasst, der das Wachstum von mindestens einem der Bakterienstämme selektiv fördert, und/oder das Agrarmedium eines Teils der Kulturbehälter weiter mindestens einen Zusatz umfasst, der das Wachstum von mindestens einem der Bakterienstämme selektiv hemmt.

3. Verfahren nach einem der Ansprüche 1 oder 2, **dadurch gekennzeichnet, dass** ein Teil der Kulturbehälter unter selektiven Bedingungen inkubiert wird, die für das Wachstum von mindestens einem der Bakterienstämme förderlich sind, und ein anderer Teil der Kulturbehälter unter unterschiedlichen selektiven Bedingungen inkubiert wird, die für das Wachstum mindestens eines anderen der Bakterienstämme förderlich sind.

4. Verfahren nach einem der Ansprüche 1 bis 3, **dadurch gekennzeichnet, dass** mindestens zwei der Bakterienstämme, die im zu prüfenden Produkt vorhanden sind, derselben Art oder Unterart angehören.

5. Verfahren nach einem der Ansprüche 1 bis 4, **dadurch gekennzeichnet, dass** mindestens einer der Bakterienstämme, die im zu prüfenden Produkt vorhanden sind, der Gattung *Lactobacillus* angehört und/oder mindestens einer der Bakterienstämme, die im zu prüfenden Produkt vorhanden sind, der Gattung *Streptococcus* angehört.

6. Verfahren nach einem der Ansprüche 1 bis 5, **dadurch gekennzeichnet, dass** das zu prüfende Produkt mindestens einen Stamm *Lactobacillus paracasei* subsp. *paracasei*, mindestens einen Stamm *Lactobacillus delbrueckii* subsp. *bulgaricus* und mindestens einen Stamm *Streptococcus thermophilus* enthält.

7. Verfahren nach Anspruch 6, **dadurch gekennzeichnet, dass** das zu prüfende Produkt weiter mindestens einen Stamm *Lactobacillus rhamnosus* enthält.

8. Verfahren nach einem der Ansprüche 6 oder 7, **dadurch gekennzeichnet, dass** zum Zählen der Bakterien der Arten *Lactobacillus paracasei* subsp. *paracasei*, *Lactobacillus rhamnosus* und *Streptococcus thermophilus* ein Medium M1 verwendet wird, dessen Zusammensetzung die Folgende ist: Agar: 15 g/L; Trypton: 2,5 g/L; pepsisches Fleischpepton: 2,5 g/L; papainisches Sojapepton: 5 g/L; Natriumglycerophosphat: 19 g/L; Laktose: 5 g/L; Hefeextrakt: 2,5 g/L; Fleischextrakt: 5 g/L; Magnesiumsulfat: 0,25 g/L; Ascorbinsäure: 0,5 g/L; 6-Chloro-3-indoxyl-beta-D-galactopyranosid (salmon Gal): 0,2 g/L; 5-Bromo-4-chloro-3-indolyl-beta-D-glucopyranosid (X-glu): 0,1 g/L.

9. Verfahren nach einem der Ansprüche 7 oder 8, **dadurch gekennzeichnet, dass** zum Zählen der Bakterien der Art *Lactobacillus delbrueckii bulgaricus* ein Medium M2 verwendet wird, dessen Zusammensetzung die Folgende ist: Polypepton: 10 g/L; Hefeextrakt: 5 g/L; Fleischextrakt: 10 g/L; Dikaliumphosphat: 2 g/L; Natriumacetat: 5 g/L; Ammoniumcitrat: 2 g/L; Magnesiumsulfat: 0,2 g/L; Mangansulfat: 0,05 g/L; Agar: 15 g/L; 5-Bromo-4-chloro-3-indolyl-beta-D-galactopyranosid (X-Gal): 0,15 g/L.

10. Verfahren nach Anspruch 8, **dadurch gekennzeichnet, dass** zum Zählen der Bakterien der Arten *Lactobacillus paracasei* subsp. *paracasei*, *Lactobacillus rhamnosus* und *Streptococcus thermophilus* ein erster Teil der Behälter, das Medium M1 enthaltend, keinen Zusatz umfasst, ein zweiter Teil der Behälter als Zusatz Vancomycin umfasst, und ein dritter Teil der Behälter als Zusatz Rhamnose umfasst.

11. Verfahren nach einem der Ansprüche 7 bis 10, **dadurch gekennzeichnet, dass** zum Zählen der Bakterien der Arten *Lactobacillus paracasei* subsp. *paracasei*, *Lactobacillus rhamnosus* und *Streptococcus thermophilus* die beimpften Behälter während etwa 48 Stunden bei kontrollierter Atmosphäre inkubiert werden, die von 6 bis 16 % O₂ und von 2 bis 10 % CO₂ enthält, wobei ein Teil der Behälter bei etwa 37 °C und ein anderer Teil bei etwa 44 °C inkubiert werden.

12. Verfahren nach einem der Ansprüche 7 bis 10, **dadurch gekennzeichnet, dass** zum Zählen der Bakterien der Art *Lactobacillus delbrueckii bulgaricus* die beimpften Behälter während etwa 48 Stunden bei etwa 47 °C bei kontrollierter Atmosphäre, die weniger als 1 % O₂ und mindestens 13 % CO₂ enthält, inkubiert werden.

## Claims

1. Method for distinguishing between and enumerating strains of lactic acid bacteria or Bifidobacteria in a known mixture, present at different population levels in a dairy product, **characterised in that** it comprises:
a) the inoculation of aliquots of said food product in a series of culture dishes each containing a chemically-defined agar culture medium M1 and at least two chromogenic substrates producing different colorations, with each one of said substrates being assimilated by at least one of said bacterial strains, and not being assimilated by at least one other of said strains; and
b) optionally, the inoculation of aliquots of said food product in a series of culture dishes containing a chemically-defined agar culture medium M2 allowing for the growth of the strains of lactic acid bacteria present in the product to be tested which cannot grow on the medium M1, and at least one chromogenic substrate assimilated by at least one of said bacterial strains; and
c) the incubation of said dishes for the time required to form bacterial colonies, and the enumeration of the colonies for each one of the colorations observed in each culture dish.

2. Method according to claim 1, **characterised in that** the agar medium of one portion of the culture dishes further comprises at least one additive that selectively favours the growth of at least one of said bacterial strains, and/or the agar culture medium of one portion of the culture dishes further comprises at least one additive that selectively inhibits the growth of at least one of said bacterial strains.

3. Method according to any one of claims 1 or 2, **characterised in that** a portion of said culture dishes is incubated in selective conditions that are favourable to the growth of at least one of said bacterial strains, and another portion of the culture dishes is incubated in different selective conditions, favourable to the growth of at least one other of said bacterial strains

4. Method according to any one of claims 1 to 3, **characterised in that** at least two of the bacterial strains present in the product to be tested belong to the same species and subspecies.

5. Method according to any one of claims 1 to 4, **characterised in that** at least one of the bacterial strains present in the product to be tested belongs to the *Lactobacillus* genus and/or at least one of the bacterial strains present in the product to be tested belongs to the *Streptococcus* genus

6. Method according to any one of claims 1 to 5, **characterised in that** the product to be tested contains at least one strain of *Lactobacillus paracasei* subsp. *paracasei*, at least one strain of *Lactobacillus delbrueckii* subsp. *bulgaricus,* and at least one strain of *Streptococcus thermophilus.*

7. Method according to claim 6, **characterised in that** the product to be tested further contains at least one strain of *Lactobacillus rhamnosus.*

8. Method according to any one of claims 6 or 7, **characterised in that** in order to enumerate the bacteria of the *Lactobacillus paracasei* subsp. *paracasei*, *Lactobacillus rhamnosus,* and *Streptococcus thermophilus* species, a medium M1 is used of which the composition is as follows: agar: 15 g/L; tryptone: 2.5 g/L; meat peptic peptone: 2.5 g/L; soy papainic peptone: 5 g/L; sodium glycerophosphate: 19 g/L; lactose: 5 g/L; yeast extract: 2.5 g/L; meat extract: 5 g/L; magnesium sulphate: 0.25 g/L; ascorbic acid: 0.5 g/L; 6-Chloro-3-indoxyl-beta-D-galactopyranoside (salmon-Gal): 0.2 g/L; 5-bromo-4-chloro-3-indolyl-beta-D-glucopyranoside (X-glu): 0.1 g/L.

9. Method according to any one of claims 7 or 8, **characterised in that** in order to enumerate the bacteria of the *Lactobacillus delbrueckii bulgaricus* species, a medium M2 is used of which the composition is as follows: polypeptone: 10 g/L; yeast extract: 5 g/L; meat extract: 10 g/L; dipotassium phosphate: 2 g/L; sodium acetate: 5 g/L; ammonium citrate: 2 g/L; magnesium sulphate: 0.2 g/L; manganese sulphate: 0,05 g/L; agar: 15 g/L; 5-Bromo-4-Chloro-3-Indolyl-Beta-D-Galactopyranoside (X-Gal): 0.15 g/L.

10. Method according to claim 8, **characterised in that** in order to enumerate the bacteria of the *Lactobacillus paracasei* subsp. *paracasei*, *Lactobacillus rhamnosus,* and *Streptococcus thermophilus* species, a first portion of the dishes containing the medium M1 does not contain any additive, a second portion of said dishes comprises, as an additive, vancomycin, and a third portion of said dishes comprises, as an additive, rhamnose.

11. Method according to any one of claims 7 to 10, **characterised in that** in order to enumerate the bacteria of the *Lactobacillus paracasei* subsp. *paracasei*, *Lactobacillus rhamnosus,* and *Streptococcus thermophilus* species, the inoculated dishes are incubated for about 48 hours in a controlled atmosphere containing from 6 to 16% O₂ and from 2 to 10% of CO₂, a portion of said dishes being incubated at about 37°C, and another portion at about 44°C.

12. Method according to any one of claims 7 to 10, **characterised in that** in order to enumerate the bacteria of the *Lactobacillus delbrueckii bulgaricus* species, the inoculated dishes are incubated for about 48 hours at about 47°C in a controlled atmosphere containing less than 1% O₂ and at least 13% CO₂.
